# EUROPÄISCHE PATENTSCHRIFT

(11) **EP 2 633 838 B1**
(45) Veröffentlichungstag und Bekanntmachung des Hinweises auf die Patenterteilung: **29.04.2020**
(21) Anmeldenummer: 13157162.2
(22) Anmeldetag: 28.02.2013
(51) Int. Cl.: A61F 9/00

(54) **Haltevorrichtung für eine Membran für eine medizinische Anwendung am Auge und Aufspannvorrichtung dazu**
Holding device for a membrane for a medical application on the eye and tensioning device for same
Dispositif de maintien pour une membrane pour une application médicale sur l''il et dispositif de tension correspondant

(30) Priorität: 02.03.2012 DE 202012100744 U
(43) Veröffentlichungstag der Anmeldung: 04.09.2013
(73) Patentinhaber: Deutsche Gesellschaft für Gewebetransplantation - gemeinnützige Gesellschaft mbH, 30625 Hannover (DE)
(72) Erfinder: Körber, Heinz, 01324 Dresden (DE)
(74) Vertreter: Sperling, Thomas

(56) Entgegenhaltungen:
- WO-A1-2006/058443
- WO-A2-03/077794
- DE-A1-102006 019 017

## Beschreibung

Die Erfindung betrifft eine Haltevorrichtung für eine Membran für eine medizinische Anwendung am Auge und eine Aufspannvorrichtung zur Positionierung der Membran in der Haltevorrichtung.

Membranen werden beispielsweise bei Behandlungen von Augenoberflächen auf diese aufgebracht und dabei am beziehungsweise auf dem Auge fixiert.

Es ist bekannt, Membranen zur Wundheilung auf der Augenoberfläche durch Verkleben oder durch Vernähen anzuordnen. Diese Art der Befestigung kann zu Problemen führen, da die Befestigung der Membran in der Regel in von der erforderlichen Wundheilung nicht betroffenen, gesunden Bereichen der Augenoberfläche erfolgt und in diesen beschwerdefreien Bereichen in Einzelfällen zu Reizungen, Blutungen, Vernarbungen oder Ähnlichem führen kann. Des Weiteren kann es durch den Lidschlag zu Kräften auf die auf der Augenoberfläche platzierte Membran kommen, die zu einer Verschiebung oder gar zu einem Abreißen der Membran mit der Notwendigkeit einer neuerlichen Befestigung führen können. Weiter bekannt ist es, eine vernetzte Membran aus einem Amnionhäutchen einzusetzen.

Alternativ zum Vernähen der Membran sind im Stand der Technik Amnionmembranhalter, beispielsweise aus der WO 03/077 794 A2, bekannt. Dabei wird die Amnionmembran mittels verschieden konstruierter Haltevorrichtungen positioniert und für den Einsatz am Auge vorbereitet. Insbesondere wird vorgeschlagen, die Membran zwischen einem inneren und einem äußeren Ring aufzuspannen, wobei der äußere Ring in eine nach innen gerichtete Nut des inneren Ringes eingespannt ist.

Alternative Befestigungen der Membran am Membranhalter werden durch eine Fixierung der Membran mittels Klemmung in einem Ring mit Spalt oder durch das Vernähen des Ringes mit der Membran realisiert.

Aus der DE 10 2006 019 017 A1 geht eine Haltevorrichtung für eine Membran und ein Träger für die Haltevorrichtung hervor, wobei die Haltevorrichtung aus zwei konzentrisch aneinander angeordneten Ringen besteht, wobei ein Innenring in einem Außenring aufgenommen wird und die beiden Ringe eine gemeinsame kegelstumpfförmige Innenfläche aufweisen, welche am Auge aufliegt.

Des Weiteren ist in der WO 2006/058443 A1 eine Vorrichtung zum Schutz des Auges bei einer Laserbehandlung offenbart. Bei einer derartigen Laserbehandlung wird die Bestrahlungsvorrichtung an der Augenlinse anliegend positioniert, wobei eine Membran die Linse vor einer Beschädigung schützt und ein Saugring die Anordnung arretiert.

Es ist eine Aufgabe der Erfindung, eine Haltevorrichtung für eine Membran und eine Aufspannvorrichtung zur Positionierung der Membran in der Haltevorrichtung zur Verfügung zu stellen, welche konstruktiv einfach aufgebaut und sicher bedienbar ist, bei reduzierten Kosten und größtmöglicher Verträglichkeit der Materialien für die medizinische Anwendung am Auge.

Die Aufgabe der Erfindung wird durch eine Haltevorrichtung und eine Aufspannvorrichtung mit den Merkmalen der selbstständigen Patentansprüche gelöst, Weiterbildungen sind in den abhängigen Unteransprüchen angegeben.

Die Aufgabe der Erfindung wird insbesondere durch eine Haltevorrichtung für eine Membran für eine medizinische Anwendung am Auge gelöst, welche einen Innenring aus einem starren Material und einen Außenring aus einem elastischen Material aufweist, zwischen denen die Membran gehaltert ist. Der Innenring wird unter Spannung von einem Außenring in einer ringnutartigen Innenringaufnahme vollständig aufgenommen. Die Innenringaufnahme erstreckt sich von innen nach außen in den Außenring hinein und der Außenring umschließt den Innenring nach Außen in einem Kontaktbereich formschlüssig korrespondierend, sodass die Membran im Kontaktbereich zwischen dem Innenring und dem Außenring angeordnet und fixiert gehaltert ist.

Besonders bevorzugt ist der Außenring aus Silikon und der Innenring aus Stahldraht ausgebildet. Insbesondere für die Anwendung der Haltevorrichtung am Auge ist es von Vorteil, dass der Innenring derart von der Innenringaufnahme aufgenommen ist, dass der Innenring keinen Kontakt mit dem Auge hat und lediglich der Außenring in Kontakt mit den Auge steht.

Besonders vorteilhaft wird der Innenring aus einem Edelstahldraht mit der Materialbezeichnung WS1.4310 ausgebildet, dessen Enden miteinander verschweißt und besonders bevorzugt laserverschweißt sind.

Nach einer vorteilhaften Ausgestaltung der Erfindung ist der Außenring im Querschnitt keilförmig nach außen spitz zulaufend. Die innere Flanke liegt am Auge an und auf die äußere Flanke läuft das Augenlied bei einem Lidschlag auf. Die keilförmige Ausgestaltung des Außenringes und somit das keilförmige Auslaufen erhöht den Tragekomfort der Vorrichtung, weil das Augenlied besser über den keilförmigen Außenring gleiten kann.
Die ringnutartige Innenringaufnahme erstreckt sich radial und horizontal von der inneren Begrenzung des Außenringes nach außen und bildet an der äußeren Begrenzung eine Hohlkehle, die in ihren Abmessungen beziehungsweise in ihrem Durchmesser mit dem Durchmesser des Innenringes korrespondiert.

Um die Haltevorrichtung mit der Membran und insbesondere der Amnionmembran zu bestücken, wird eine Aufspannvorrichtung für eine Membran erfindungsgemäß vorgeschlagen, mit deren Hilfe die Positionierung der Membran in der Haltevorrichtung rationell manuell oder auch maschinell automatisiert erfolgen kann.
Die Aufspannvorrichtung besteht dabei im Wesentlichen aus zwei Teilen, einem Oberteil zur temporären Aufnahme des Innenringes und einem Unterteil zur temporären Aufnahme des Außenringes der Haltevorrichtung.
Oberteil und Unterteil sind relativ zu einander beweglich und werden zur Aufspannung der Membran aufeinander aufgesetzt. Die Membran ist zwischen dem Oberteil und dem Unterteil positionierbar und es sind weiterhin Mittel vorgesehen, die den Innenring während des Aufspannvorganges der Membran unter Mitnahme der Membran in die korrespondierende Innenringaufnahme des Außenringes führen. Dabei wird die Membran zwischen Innenring und Außenring kraft- und reibschlüssig im Kontaktbereich zwischen dem Innenring und dem Außenring eingespannt und fixiert.

Nach einer vorteilhaften Ausgestaltung der Erfindung weist das Oberteil der Aufspannvorrichtung einen oberen Stempel zur Aufnahme des Innenringes auf und besitzt einen relativ zum oberen Stempel axial beweglichen Positionierkonus. Der Positionierkonus ist dazu ausgebildet, den Innenring vom oberen Stempel abzustreifen und den Innenring in die Innenringaufnahme des gegenüberliegend am Unterteil der Aufspannvorrichtung platzierten Außenringes hineinzuschieben und zu führen.

Bevorzugt ist der Positionierkonus über einen Druckring betätigbar und axial verschiebbar gegenüber dem Oberteil ausgebildet.

Die Aufspannvorrichtung besitzt vorteilhaft einen Druckstempel, der den oberen Stempel betätigt und wie der obere Stempel axial verschiebbar gegenüber dem Oberteil ausgebildet ist. Korrespondierend dazu ist der untere Stempel des Unterteils gleichfalls axial bewegbar ausgebildet, sodass der auf dem unteren Stempel aufgezogene Außenring bei einer Betätigung des Druckstempels und einer Bewegung des oberen Stempels und des an diesen angrenzenden unteren Stempels vom unteren Stempel gegen einen Abstreifkonus gedrückt und sich dabei um den Innenring verdrehend unter Klemmung der Membran zwischen dem Außenring und dem Innenring abstreifbar ausgebildet ist.

Vorteilhaft ist der untere Stempel der Form des Augapfels konvex nachgebildet und der obere Stempel korrespondierend dazu konkav ausgebildet.

Nach einer besonders vorteilhaften Ausgestaltung der Aufspannvorrichtung ist das Oberteil und das Unterteil drehgelenkig miteinander verbunden, wobei in einer Aufziehstellung der obere und untere Stempel, der Positionierkonus sowie der Abstreifkonus in einer Achse liegen und in Bestückstellung zur leichteren Bestückung der Aufspannvorrichtung mit Einzelteilen der Haltevorrichtung und zur Entnahme der montierten Haltevorrichtung das Oberteil vom Unterteil wegschwenkbar ausgebildet ist.

Die Vorteile der Erfindung bestehen insbesondere darin, dass der Innenring derart vollständig vom Außenring aufgenommen wird, dass dieser nicht in Kontakt mit dem Auge kommt. Der Außenring aus flexiblem und medizinisch gut verträglichem Material umschließt den Innenring in Form und Maß an der Außenseite sowie oben und unten vollständig und die Innenseite des Innenringes ist so weit in der Innenringaufnahme versenkt, dass der Innenring keinen Kontakt mit dem Auge hat. Die Halterung der Membran wird in einem Kontaktbereich innerhalb der ringnutartigen Innenringaufnahme realisiert.

Die Komponenten der Haltevorrichtung sind kostengünstig produzierbar und die Aufspannvorrichtung zur Montage von Innenring und Außenring mit der Membran ist einfach bedienbar.

Weitere Einzelheiten, Merkmale und Vorteile der Erfindung ergeben sich aus der nachfolgenden Beschreibung von Ausführungsbeispielen mit Bezugnahme auf die zugehörigen Zeichnungen. Es zeigen:
- Fig. 1:: Aufspannvorrichtung
- Fig. 2:: Haltevorrichtung
- Fig. 3:: Außenring im vergrößerten Querschnitt
- Fig. 4:: Außenring im Querschnitt
- Fig. 5:: Außenring in der Draufsicht

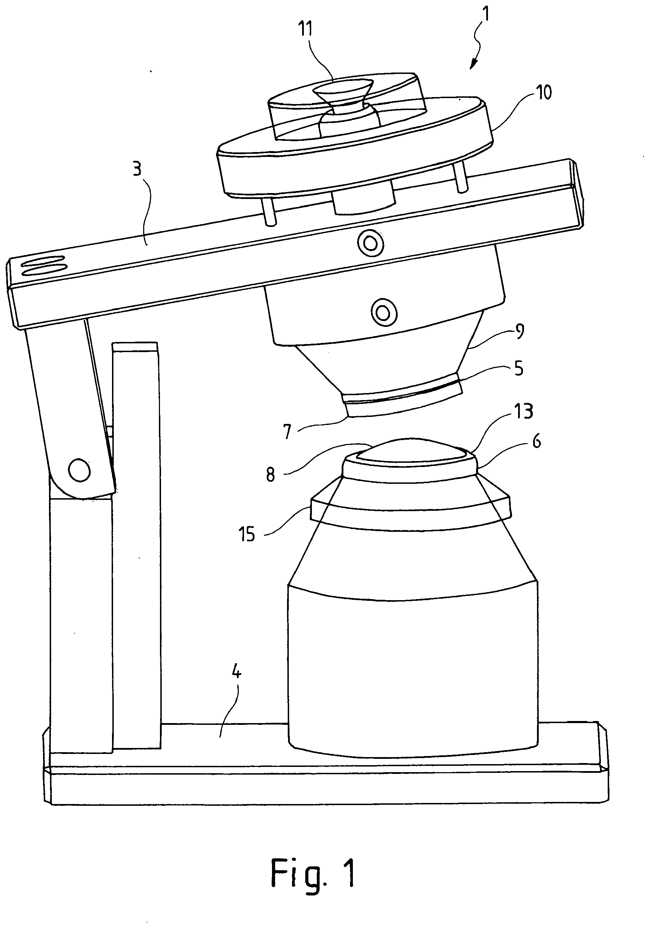

In Figur 1 ist eine Aufspannvorrichtung 1 in halb geöffneter Position dargestellt. Die Aufspannvorrichtung 1 besteht im Wesentlichen aus einem Oberteil 3 und einem Unterteil 4, welche mittels eines Gelenks miteinander verbunden sind. In der dargestellten Ausgestaltung ist das Oberteil 3 auf das Unterteil 4 durch eine drehgelenkige Verbindung aufschwenkbar. Am Oberteil 3 ist der obere Stempel 7 axial über einen Druckstempel 11 verschiebbar angeordnet. Die Betätigung des Druckstempels 11 führt zu einer axialen Verschiebung des oberen Stempels 7 relativ zum rahmenartigen Oberteil 3.
Weiterhin ist ein Positionierkonus 9 gleichfalls axial verschiebbar am Oberteil 3 vorgesehen, welcher über einen axial bewegbaren Druckring 10 betätigbar ausgebildet ist.
Das Unterteil 4 der Aufspannvorrichtung 1 weist im Wesentlichen einen unteren Stempel 8, der gleichfalls axial bewegbar ausgebildet ist, auf und besitzt zudem einen Abstreifkonus 15, welcher relativ zum Unterteil 4 nicht beweglich ausgebildet ist.

Die Handhabung der Aufspannvorrichtung 1 erfolgt, indem zunächst in aufgeklappten Zustand der Innenring 5 aus Edelstahldraht auf den oberen Stempel 7 des Oberteils 3 aufgezogen wird. Der obere Stempel 7 ist dazu zylindrisch beziehungsweise leicht konisch oder angefast ausgebildet, sodass der Innenring 5 ohne zusätzliche Hilfsmittel von Hand auf den Stempel 7 aufgezogen beziehungsweise aufgeschoben werden kann. Um den Innenring 5 auf dem oberen Stempel 7 zu halten, ist eine kleine Drahtfeder in einer Längsnut des oberen Stempels 7 vorgesehen, die den aufgeschobenen Innenring 5 mit leichter Spannung in radialer Richtung auf dem oberen Stempel 7 hält. Nun wird der Außenring 6 aus Silikon mit der konischen schmalen Seite nach unten auf den unteren Stempel 8 aufgesetzt und die Spitze nach außen abkragende Lippe des im Querschnitt keilförmigen Außenringes 6 nach unten umgestülpt. Dadurch ist die nutartige Innenringaufnahme 13 vertikal ausgerichtet und zeigt mit der offenen Seite nach oben und ist bereit für das Einführen des Innenringes 5 in die Innenringaufnahme 13 von oben. Nunmehr wird die Membran auf den unteren Stempel 8 mit der nicht mit dem Auge in Kontakt kommenden Außenseite nach unten aufgelegt, wobei die Membran den Stempel 8 und die Innenringaufnahme 13 in radialer Richtung überlappt.

Bei einer derartig präparierten Aufspannvorrichtung 1 wird nun das Oberteil 3 heruntergeklappt, wobei der obere Stempel 7, der untere Stempel 8 sowie der Positionierkonus 9, der Druckring 10 und der Druckstempel 11 in einer Achse fluchtend positioniert werden.

Die Membran ist nun zwischen dem oberen Stempel 7 und dem unteren Stempel 8 positioniert.
Nachfolgend wird der Druckring 10 nach unten bewegt, wodurch sich der Positionierkonus 9 relativ zum oberen Stempel 7 bewegt und dabei den Innenring 5 erfasst und in die darunter liegende Nut der Innenringaufnahme 13 des Außenringes 6 führt, wobei die dazwischenliegende Membran mitgeführt und zwischen dem in der Innenringaufnahme 13 aufgenommenen Innenring 5 und dem Außenring 6 festklemmend gehaltert wird. Nunmehr wird zusätzlich zum Druckring 10 der Druckstempel 11 betätigt, wodurch der obere Stempel 7 gemeinsam mit dem unteren Stempel 8 axial nach unten bewegt werden und der auf dem unteren Stempel 8 aufliegende Außenring 6 wird gegen den Abstreifkonus 15 gedrückt, was dazu führt, dass der Außenring 6 nach oben mit seinem keilförmigen Ende zurückklappt und dabei den Innenring 5 mit der Membran fest in der Innenringaufnahme 13 des Außenringes 6 unter Spannung fixiert.

Die keilförmige Lippe des Außenringes 6 schnappt wie beschrieben nach oben zurück und die Membran ist fertig eingespannt. Hiernach wird das Oberteil 3 vom Unterteil 4 weggeschwenkt und die fertig präparierte Haltevorrichtung, bestehende aus Innenring 5, Außenring 6 und der Membran, zur Entnahme aus der Aufspannvorrichtung 1 frei gegeben.
Die Membran 12 überspannt den gesamten Durchmesser des Innenrings 5, wird zwischen dem Innenring 5 und dem Außenring 6 gehalten und legt sich, soweit überstehend, an den Außenring 6 an der inneren Flanke an und liegt somit zwischen dem Auge und dem Außenring 6, welcher bei entsprechendem Überstand der Membran keinen Kontakt mit dem Auge hat. Dies ist besonders vorteilhaft, um materialbedingte Irritationen der Augenoberfläche zu vermeiden.

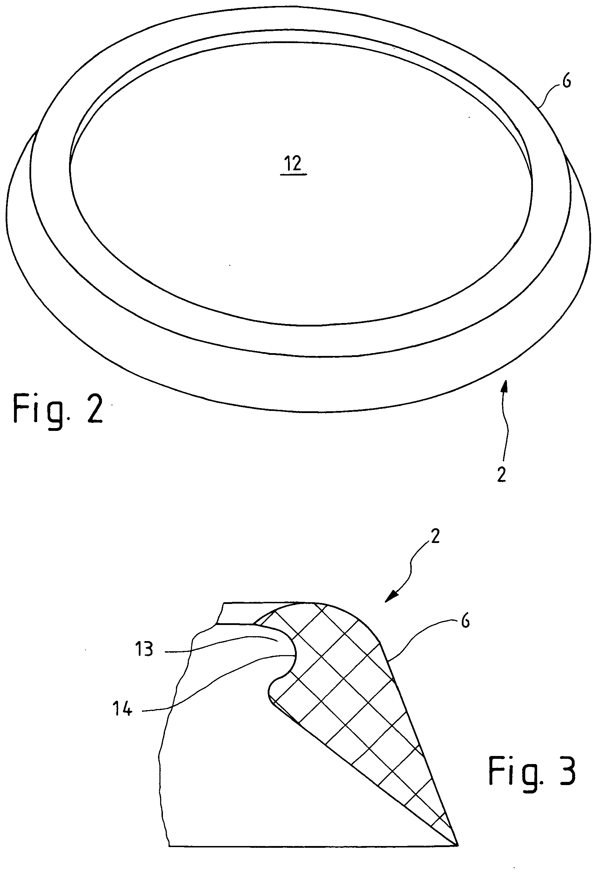

In der Figur 2 ist eine Haltevorrichtung 2 in dreidimensionaler Ansicht dargestellt, welche die aufgespannte Membran 12 und den Außenring 6 zeigt. Der Innenring 5 ist fast vollständig vom Außenring 6 verdeckt und nur im oberen Teil der Abbildung zu sehen. Die äußere und die innere Kontur des Außenringes 6 sind konisch ausgeführt, wodurch sich der keilförmige Querschnitt des Außenringes 6 ergibt. Die obere Begrenzung des Außenringes 6 ist abgerundet ausgestaltet, um einen größtmöglichen Tragekomfort der Haltevorrichtung 2 auf dem Auge zu erreichen.

In Figur 3 ist der Querschnitt des Außenringes 6 der Haltevorrichtung 2 vergrößert dargestellt. Das keilförmige Zulaufen der Schenkel im unteren Bereich dient der Erhöhung des Tragekomforts der Haltevorrichtung 2 bei der medizinischen Anwendung am Auge.
Im oberen Bereich weist der Querschnitt des Außenringes 6 eine sich radial nach außen erstreckende ringnutartige Innenringaufnahme 13 auf, die nach außen durch den Kontaktbereicht 14 begrenzt ist. Die untere Flanke des Keiles und innere Fläche des Außenringes 6 liegt auf dem Auge auf und passt sich der jeweiligen Kontur des Augapfels durch das hochflexible Silikonmaterial an, wobei zwischen dem Außenring 6 und dem Auge die Membran 12 bei entsprechender Dimensionierung angeordnet ist. In dieser Ausgestaltung hat weder der Innenring 5 noch der Außenring 6 direkten Kontakt mit der Augenoberfläche. Die obere Flanke des Keiles und äußere Fläche des Außenringes 6 jedoch wird bei einer Bewegung des Augenlids von diesem überstrichen und ist entsprechend verträglich dafür ausgebildet.

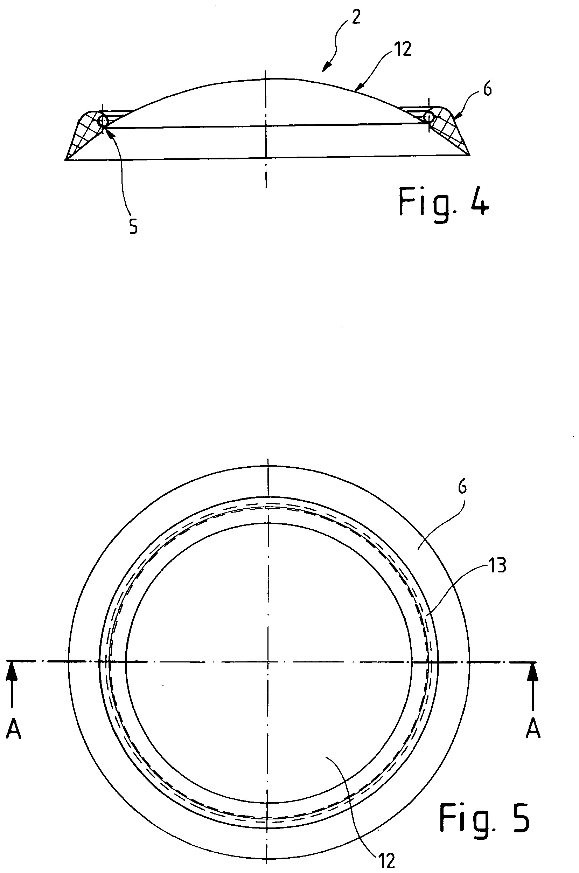

In Figur 4 ist die Haltevorrichtung 2 mit dem Außenring 6 und dem Innenring 5 sowie der Membran 12 in montierter Form in einer Seitenansicht im Querschnitt dargestellt. Die Membran 12 folgt dabei der Rundung des Auges im Bereich der Hornhaut und ist zwischen dem Innenring 5 und dem Außenring 6 im vergrößert in Figur 3 dargestellten Kontaktbereich 14 festgeklemmt.

Figur 5 zeigt eine die Figur 4 ergänzende Draufsicht des Außenringes 6 mit der mit verdeckten Linien dargestellten Innenringaufnahme 13 und der Membran 12.

### LISTE DER BEZUGSZEICHEN

- 1: Aufspannvorrichtung
- 2: Haltevorrichtung
- 3: Oberteil
- 4: Unterteil
- 5: Innenring
- 6: Außenring
- 7: oberer Stempel
- 8: unterer Stempel
- 9: Positionierkonus
- 10: Druckring
- 11: Druckstempel
- 12: Membran
- 13: Innenringaufnahme
- 14: Kontaktbereich
- 15: Abstreifkonus

## Patentansprüche

1. Haltevorrichtung (2) für eine Membran (12) für eine medizinische Anwendung am Auge, aufweisend einen Innenring (5) aus einem starren Material und einen Außenring (6) aus einem elastischen Material zwischen denen die Membran (12) gehaltert ist, der Innenring (5) unter Spannung vom Außenring (6) in einer ringnutartigen Innenringaufnahme (13) vollständig aufgenommen ist, wobei sich die Innenringaufnahme (13) von innen nach außen in den Außenring (6) hinein erstreckt und der Außenring (6) den Innenring (5) nach außen in einem Kontaktbereich (14) formschlüssig korrespondierend umschließt und dass die Membran (12) im Kontaktbereich (14) zwischen dem Innenring (5) und dem Außenring (6) angeordnet und gehaltert ist, wobei der Außenring (6) aus einem flexiblen und medizinisch gut verträglichen Material besteht, **dadurch gekennzeichnet, dass** besagter Außenring den Innenring (5) in Form und Maß an der Außenseite sowie oben und unten vollständig umschließt und die Innenseite des Innenringes (5) so weit in der Innenringaufnahme (13) versenkt ist, dass der Innenring (5) keinen Kontakt mit dem Auge hat.

2. Haltevorrichtung (2) nach Anspruch 1, **dadurch gekennzeichnet, dass** der Außenring (6) aus Silikon und der Innenring (5) aus Stahldraht ausgebildet ist.

3. Haltevorrichtung (2) nach Anspruch 1 oder 2, **dadurch gekennzeichnet, dass** der Innenring (5) aus einem Edelstahldraht ausgebildet ist dessen Enden miteinander verschweißt sind.

4. Haltevorrichtung (2) nach einem der Ansprüche 1 bis 3, **dadurch gekennzeichnet, dass** der Außenring (6) im Querschnitt nach außen sich mit einer Flanke an das Auge anlegend keilförmig ausgebildet ist, wobei die Innenringaufnahme (13) sich radial und horizontal nach außen erstreckt.

5. Aufspannvorrichtung (1) für eine Membran (12) zu deren Positionierung in einer Haltevorrichtung (2) gemäß der vorangegangenen Ansprüche, **dadurch gekennzeichnet, dass** ein Oberteil (3) zur temporären Aufnahme des Innenringes (5) und ein Unterteil (4) zur temporären Aufnahme des Außenringes (6) der Haltevorrichtung (2) relativ zueinander beweglich vorgesehen sind, wobei das Unterteil und das Oberteil konfiguriert sind so dass die Membran (12) zwischen dem Oberteil (3) und dem Unterteil (4) positionierbar ist und dass Mittel in der Aufspannvorrichtung vorgesehen sind, die konfiguriert sind den Innenring (5) während des Aufspannvorganges der Membran (12) unter Mitnahme der Membran (12) in die korrespondierende Innenringaufnahme (13) des Außenringes (6) führen, wobei die Aufspannvorrichtung so konfiguriert ist dass die Membran (12) zwischen Innenring (5) und Außenring (6) kraft- und reibschlüssig im Kontaktbereich (14) fixiert wird und wobei das Oberteil (3) einen oberen Stempel (7) zur Aufnahme des Innenrings (5) und einen relativ zum oberen Stempel (7) axial beweglichen Positionierkonus (9) aufweist, wobei die Aufspannvorrichtung derart konfiguriert ist dass der Positionierkon us (9) den Innenring (5) vom oberen Stempel (7) abstreifbar und den Innenring (5) in die Innenringaufnahme (13) des gegenüberliegend am Unterteil (4) platzierten Außenringes (6) führbar ausgebildet ist, wobei der Außenring (6) aus flexiblem und medizinisch gut verträglichem Material den Innenring (5) in Form und Maß an der Außenseite sowie oben und unten vollständig umschließt und die Innenseite des Innenringes (5) so weit in der Innenringaufnahme (13) versenkt wird, dass der Innenring (5) keinen Kontakt mit dem Auge hat.

6. Aufspannvorrichtung (1) nach Anspruch 5, **dadurch gekennzeichnet, dass** der Positionierkonus (9) über einen Druckring (10) betätigbar axial verschiebbar gegenüber dem Oberteil (3) ausgebildet ist.

7. Aufspannvorrichtung (1) nach Anspruch 5 oder 6, **dadurch gekennzeichnet, dass** der obere Stempel (7) über einen Druckstempel (11) betätigbar und axial verschiebbar gegenüber dem Oberteil (3) ausgebildet ist und dass korrespondierend dazu der untere Stempel (8) des Unterteils (4) dadurch axial bewegbar ausgebildet ist, sodass der auf dem unteren Stempel (8) aufgezogene Außenring (6) bei einer Betätigung des Druckstempels (11) und einer Bewegung des oberen Stempels (7) und des unteren Stempels (8) von diesem gegen einen Abstreifkonus (15) gedrückt und sich dabei um den Innenring (5) verdrehend unter Klemmung der Membran (12) zwischen dem Außenring (6) und dem Innenring (5) abstreifbar ausgebildet ist.

8. Aufspannvorrichtung (1) nach einem der Ansprüche 5 bis 7, **dadurch gekennzeichnet, dass** der untere Stempel (8) der Form des Augapfels konvex nachgebildet ist und dass der obere Stempel (7) korrespondierend konkav ausgebildet ist.

9. Aufspannvorrichtung (1) nach einem der Ansprüche 5 bis 8, **dadurch gekennzeichnet, dass** das Oberteil (3) und das Unterteil (4) drehgelenkig miteinander verbunden sind, wobei in einer Aufziehstellung die Stempel (7, 8, 11), der Positionierkonus (9) sowie der Abstreifkonus (15) in einer Achse liegen und in einer Bestückstellung zur leichteren Be- und Entladung das Oberteil (3) vom Unterteil (4) wegschwenkbar ausgebildet ist.

## Claims

1. Holding device (2) for a diaphragm (12) for a medical application on the eye, having an inner ring (5) made from a rigid material and an outer ring (6) made from an elastic material between which the diaphragm (12) is held, the inner ring (5) under tension from the outer ring (6) is fully accommodated in an inner ring receiving element (13) that is like an annular groove, wherein the inner ring receiving element (13) extends from inside to outside into the outer ring (6) and the outer ring (6), corresponding in a form-fit manner, encloses the inner ring (5) outwards in a contact zone (14) and that the diaphragm (12) is arranged and held in the contact zone (14) between the inner ring (5) and the outer ring (6), wherein
the outer ring (6) is made from a flexible and medically well tolerated material, **characterised in that** said outer ring completely encloses the inner ring (5) in shape and dimensions on the outer side and also at the top and bottom, and the inner side of the inner ring (5) is sunk so far into the inner ring receiving element (13) that the inner ring (5) has no contact with the eye.

2. Holding device (2) in accordance with Claim 1, **characterised in that** the outer ring (6) is made from silicone and the inner ring (5) is made from steel wire.

3. Holding device (2) in accordance with Claim 1 or 2, **characterised in that** the inner ring (5) is constructed from a stainless steel wire whose ends are welded together.

4. Holding device (2) in accordance with one of Claims 1 to 3, **characterised in that** the outer ring (6) in its cross section towards the outside is designed with a side abutting the eye in a wedge-shaped fashion, wherein
the inner ring receiving element (13) extends radially and horizontally towards the outside.

5. Clamping device (1) for a diaphragm (12) for its positioning in a holding device (2) in accordance with the preceding claims, **characterised in that** an upper part (3) for temporarily receiving the inner ring (5) and a lower part (4) for temporarily receiving the outer ring (6) of the holding device (2) are envisaged moveable relative to each other, wherein the lower part and the upper part are configured such that the diaphragm (12) is capable of being positioned between the upper part (3) and the lower part (4) and that means are provided in the clamping device which are configured, during the clamping process for the diaphragm (12), such as to guide the inner ring (5), taking with it the diaphragm (12), into the corresponding inner ring receiving element (13) of the outer ring (6), wherein the clamping device is so configured that the diaphragm (12) is fixed between inner ring (5) and outer ring (6) in the contact zone (14) in a force-fit and frictionally engaged manner, wherein
the upper part (3) has an upper plunger (7) for receiving the inner ring (5) and a positioning cone (9) that is axially moveable relative to the upper plunger (7), wherein the clamping device is configured in such a way that the positioning cone (9) is designed capable of removing the inner ring (5) from the upper plunger (7) and guiding the inner ring (5) into the inner ring receiving element (13) of the outer ring (6) positioned opposite on the lower part (4), wherein the outer ring (6) made from flexible and medically well tolerated material fully encloses the inner ring (5) in shape and dimensions and also at the top and bottom, and the inner side of the inner ring (5) is sunk so far into the inner ring receiving element (13) that the inner ring (5) has no contact with the eye.

6. Clamping device (1) in accordance with Claim 5, **characterised in that** the positioning cone (9) - operatable via a pressure ring (10) - is designed axially displaceable with respect to the upper part (3).

7. Clamping device (1) in accordance with Claim 5 or 6, **characterised in that** the upper plunger (7) is designed capable of being operated via a pressure plunger (11) and axially displaceable with respect to the upper part (3) and that, corresponding to this, the lower plunger (8) of the lower part (4) is thereby designed axially moveable, so that the outer ring (6) that has been pulled onto the lower plunger (8) is, on operation of the pressure plunger (11) and on movement of the upper plunger (7) and the lower plunger (8), is pressed by the latter against a removing cone (15) and at the same time is designed capable - twisting around the inner ring (5) with clamping of the diaphragm (12) between the outer ring (6) and the inner ring (5) - of being removed.

8. Clamping device (1) in accordance with one of Claims 5 to 7, **characterised in that** the lower plunger (8) is convex modelling the shape of the eyeball, and that the upper plunger (7) is designed concave to correspond to this.

9. Clamping device (1) in accordance with one of Claims 5 to 8, **characterised in that** the upper part (3) and the lower part (4) are connected to each other with a swivel joint, wherein in a 'pulling-on position' the plungers (7, 8, 11), the positioning cone (9) and the removing cone (15) lie in one axis und in a loading position, for easier loading and unloading, the upper part (3) is designed capable of being swivelled out of the way of the lower part (4).

## Revendications

1. Dispositif de maintien (2) d'une membrane (12) destiné à une application médicale oculaire, constitué d'une bague intérieure (5) réalisée dans un matériau rigide et d'une bague extérieure (6) réalisée dans un matériau élastique entre lesquelles est maintenue la membrane (12), la bague intérieure (5) étant entièrement prise et maintenue, de par la pression de la bague extérieure (6), dans un
logement annulaire interne (13) de type gorge, le logement annulaire interne (13) s'étendant de l'intérieur vers l'extérieur dans la bague extérieure (6) et la bague extérieure (6) entourant la bague intérieure (5) sur sa face externe dans une zone de contact
(14) parfaitement adaptée, la membrane
(12) étant logée et maintenue dans la zone de contact (14) entre la bague intérieure (5)
et la bague extérieure (6), la bague extérieure (6) étant réalisée dans un matériau souple et compatible avec une application médicale,
**caractérisé en ce que** ladite bague extérieure
recouvre entièrement et parfaitement la bague intérieure (5) sur sa face extérieure ainsi qu'en haut et en bas et **en ce que** la face interne de la bague intérieure (5) est si profondément enfoncée dans le logement annulaire (13) que la bague intérieure (5) n'entre jamais en contact avec l'œil.

2. Dispositif de maintien (2) selon la revendication 1, **caractérisé en ce que** la bague extérieure (6) est réalisée en silicone et la bague intérieure (5) est réalisée en fil métallique.

3. Dispositif de maintien (2) selon la revendication 1 ou 2, **caractérisé en ce que** la bague intérieure (5) se compose d'un fil métallique dont les extrémités sont soudées entre elles.

4. Dispositif de maintien (2) selon les revendications 1 à 3, **caractérisé en ce que** la bague extérieure (6) présente, dans sa partie extérieure, une surface cunéiforme reposant sur l'œil,
le logement annulaire (13) s'étendant de manière radiale et horizontale vers l'extérieur.

5. Dispositif de serrage (1) d'une membrane (12) aux fins de son positionnement dans un dispositif de maintien (2) conformément aux revendications précédentes, **caractérisé en ce qu'**une partie supérieure (3) prévue pour le logement temporaire de la bague intérieure (5) et une partie inférieure (4) prévue pour le logement temporaire de la bague extérieure (6) du dispositif de maintien (2) sont
mobiles l'une par rapport à l'autre, la partie inférieure et
la partie supérieure étant configurées de manière à ce que la membrane (12) soit positionnable entre la partie supérieure (3) et la partie inférieure (4) et **en ce que** des moyens prévus dans le dispositif de serrage
sont configurés de sorte que
la bague intérieure (5), pendant le
processus de serrage de la membrane (12), s'insère avec la membrane (12) dans le logement annulaire (13) correspondant de la bague externe,
(6) le dispositif de serrage étant configuré de sorte que
la membrane (12) entre la bague intérieure (5) et la
bague extérieure (6) soit maintenue par pression et par friction dans la zone de contact (14)
et que
partie supérieure (3) est dotée d'un piston supérieur (7) pour recevoir la bague intérieure (5) et un cône de positionnement (9) se déplaçant de manière axiale par rapport au piston supérieur (7), le dispositif de serrage étant configuré de telle manière que le
cône de positionnement (9)
se compose de la bague intérieure (5) extractible du piston supérieur (7) et de la bague inférieure (5) dans le logement annulaire (13) de la bague extérieure (6) placée sur la partie inférieure (4) opposée, la bague extérieure (6) en matériau flexible et compatible avec un usage médical enveloppant parfaitement la bague intérieure (5) sur sa face extérieure mais aussi en haut et en bas et la face interne de la bague intérieure (5) étant si profondément insérée dans le logement annulaire (13) que
la bague intérieure (5) n'entre jamais en contact avec l'œil.

6. Dispositif de serrage (1) selon la revendication 5, **caractérisé en ce que** le cône de positionnement (9) peut être actionné axialement à l'aide d'une bague de pression (10) mobile par rapport à la partie supérieure (3).

7. Dispositif de serrage (1) selon la revendication 5 ou 6, **caractérisé en ce que** le piston supérieur (7) comprend un piston de compression (11) (11) actionnable et mobile axialement par rapport à la partie supérieure (3) et **en ce que** le piston inférieur (8) correspondant de la partie inférieure (4) est mobile axialement, de sorte que la bague extérieure (6) posée sur le piston inférieur (8), grâce à l'actionnement du piston de compression (11) et au déplacement du piston supérieur (7) et du piston inférieur (8), est compressée par ce dernier sur un cône d'extraction (15) et se déforme autour de la bague intérieure (5) en serrant la membrane (12) entre la bague extérieure (6) et la bague intérieure (5).

8. Dispositif de serrage (1) selon l'une des revendications 5 à 7, **caractérisé en ce que** le piston inférieur (8) a la forme convexe d'un globe oculaire et **en ce que** le piston inférieur (7) correspondant est concave.

9. Dispositif de serrage (1) selon l'une des revendications 5 à 8, **caractérisé en ce que** la partie supérieure (3) et la partie inférieure (4) sont liées entre elles par une articulation rotative, les pistons (7, 8, 11) le cône de positionnement (9) et le cône d'extraction (15) étant positionnés en ligne au niveau du poste d'utilisation et de manière à basculer pour faciliter le chargement et le déchargement de la partie supérieure (3) au niveau de la partie inférieure (4).
